# EUROPÄISCHE PATENTSCHRIFT

(11) **EP 1 227 762 B1**
(45) Veröffentlichungstag und Bekanntmachung des Hinweises auf die Patenterteilung: **03.03.2004**
(21) Anmeldenummer: 00977502.4
(22) Anmeldetag: 07.11.2000
(51) Int. Cl.: A61B 17/16

(54) **CHIRURGISCHES BEARBEITUNGSWERKZEUG ZUR SCHAFFUNG EINER VERTIEFUNG IN EINEM KNORPEL- UND/ODER KNOCHENGEWEBE FÜR EINE GELENKSPROTHESE**
SURGICAL TREATMENT TOOL FOR CREATING A RECESS IN A CARTILAGE AND/OR BONE TISSUE FOR A JOINT PROSTHESIS
OUTIL DE TRAITEMENT CHIRURGICAL DESTINE A PRATIQUER UNE CAVITE DANS UN CARTILAGE ET/OU TISSU OSSEUX POUR UNE PROTHESE ARTICULAIRE

(30) Priorität: 08.11.1999 DE 19953611
(43) Veröffentlichungstag der Anmeldung: 07.08.2002
(73) Patentinhaber: Szabo, Zsolt, D-80933 München (DE); Müller, Erich Johann, Dr. med., 63839 Kleinwallstadt (DE)
(72) Erfinder: Szabo, Zsolt, 80933 München (DE)
(74) Vertreter: Kurig, Thomas, Dr. Dipl.-Phys.
(86) Internationale Anmeldenummer: PCT/EP2000/010985
(87) Internationale Veröffentlichungsnummer: WO 2001/034039

(56) Entgegenhaltungen:
- GB-A- 2 321 598
- US-A- 2 785 673
- US-A- 3 630 204
- US-A- 5 203 653
- US-A- 5 376 092

## Beschreibung

Die Erfindung betrifft ein chirurgisches Bearbeitungswerkzeug zur Schaffung einer Vertiefung in einem Knorpel- und/oder Knochengewebe für eine Gelenksprothese, insbesondere in einem Hüftgelenksknochen für eine Pfannenprothese, welches eine im wesentlichen halbkugelförmige Mantelfläche und einen dadurch umgrenzten hohlen Innenbereich aufweist, wobei an der Mantelfläche zumindest eine sich vom Pol der Mantelfläche zum Äquator der Mantelfläche erstreckende Schneide, welche zumindest ein Schneidelement (20) mit einer dem Werkstück zugewandten Schneidfläche (26) und einer dem Werkstück abgewandten Schneidfläche (28) aufweist, und eine damit kooperierende Öffnung in der Mantelfläche zum Abtransport des Spans in den hohlen Innenbereich ausgebildet sind, wobei zur Drehung um die die Drehachse bildende Symmetrieachse an der Mantelfläche ein sich durch den hohlen Innenbereich erstreckender Lagerschaft vorteilhaft befestigt ist.

Ein derartiges chirurgisches Bearbeitungswerkzeug ist aus der WO 95/13749 bekannt, welche ein Bearbeitungswerkzeug zur Ausbildung einer Vertiefung in einer Hüftgelenkspfanne offenbart, welches einen halbkugelförmigen Bearbeitungskopf aufweist. Dieser Bearbeitungskopf weist mehrere Schneiden und damit zusammenwirkende Öffnungen auf, durch welche das abzutragende Material in einen hohlen Innenbereich abtransportiert wird, so daß der Bearbeitungsvorgang nicht aufgrund von abgetragenem Material unterbrochen werden muß.

Ferner offenbart die EP 0574701 A1 ein Bearbeitungswerkzeug für Knochen, welches einen halbkugelförmigen Kopf aufweist, welcher mitsamt der Schneiden aus Kunststoff hergestellt ist. Dies ermöglicht eine besonders kostengünstige Herstellung des Werkzeugs, wodurch eine Einmalanwendung möglich ist. Darüber hinaus offenbart diese Druckschrift eine Ausführungsform eines Bearbeitungswerkzeugs, die eine spiralförmig den Schaft umlaufende Schneide aufweist, welche aus einer Vielzahl von Schneidelementen aufgebaut ist.

Des weiteren offenbart die EP 0782840 A1 ein chirurgisches Fräswerkzeug zum Einsetzen von Prothesen, welches einen halbkugelförmigen Bearbeitungskopf und einen mittels eines Anschlußstücks zu verbindenden Lagerschaft aufweist, wobei der Bearbeitungskopf ein zentrales Schneidelement zur Führung des Fräswerkzeugs und eine Vielzahl von über dem halbkugelförmigen Bearbeitungskopf verteilten Schneidelementen aufweist.

Derartige Bearbeitungswerkzeuge weisen jedoch den Nachteil auf, daß der halbkugelförmige Werkzeugkopf zum Rand bzw. zum Äquator hin aufgrund der größeren Schneidgeschwindigkeit einen immer höheren Materialabtrag aufweist. Dadurch weicht die geometrische Form der Vertiefung von der für die Prothese benötigte halbkugelförmige Ausgestaltung ab. Folglich kommt es zu einer Paßungenauigkeit zwischen der Vertiefung und der darin aufgenommenen Pfannenprothese. Dies führt zu kürzeren Wechselintervallen der Prothese, so daß sich der Patient in relativ kurzen Abständen einer Operation unterziehen muß, um eine neue Prothese eingesetzt zu bekommen.

Daher ist es die Aufgabe der vorliegenden Erfindung, ein chirurgisches Bearbeitungswerkzeug zur Ausbildung einer Vertiefung in einem Knorpel und/oder Knochengewebe, insbesondere in einem Hüftgelenksknochen für eine Pfannenprothese, zu schaffen, durch welches eine Vertiefung mit einer möglichst exakten geometrischen Form herstellbar ist.

Diese Aufgabe wird mittels eines chirurgischen Bearbeitungswerkzeugs gemäß dem Oberbegriff des Anspruchs 1 dadurch gelöst, daß ein Freiwinkel γ des Schneidelements, welcher zwischen der Mantelfläche und der dem Werkstück zugewandten Schneidfläche ausgebildet ist, vom Pol der Mantelfläche in Richtung des Äquators der Mantelfläche abnimmt.

Durch die Abnahme des Freiwinkels γ wird der Materialabtrag zum Rand bzw. Äquator der Halbkugel hin vermindert, so daß die geometrische Form der Vertiefung nahezu exakt der Form des halbkugelförmigen Bearbeitungswerkzeugs entspricht. Aufgrund der exakten Ausbildung der halbkugelförmigen Vertiefung kommt es zu einer besseren Paßgenauigkeit zwischen der Pfannenprothese und der Vertiefung, so daß sich die Lebensdauer der eingesetzten Prothese erhöht. Folglich verlängern sich die Intervalle zwischen den Operationen, bei welchen dem Patienten eine neue Prothese eingesetzt werden muß.

Ferner wird durch die Abnahme des Freiwinkels zum Rand des Bearbeitungswerkzeugs hin die Tendenz des Verlaufens des erfindungsgemäßen Bearbeitungswerkzeuges gegenüber herkömmlichen Bearbeitungswerkzeugen vermindert, da die maximale Schneidleistung des erfindungsgemäßen Bearbeitungswerkzeuges gegenüber herkömmlichen Bearbeitungswerkzeugen näher am Pol der Mantelfläche angeordnet ist. Dies führt dazu, daß das erfindungsgemäße Bearbeitungswerkzeug gegenüber herkömmlichen Bearbeitungswerkzeugen bei entsprechendem Vorschub in Richtung der Drehachse während des Bearbeitungsvorgangs selbstzentrierend ist.

Weitere vorteilhafte Ausgestaltungen der Erfindung sind Gegenstand der Unteransprüche.

Durch eine Weiterbildung ist die Herstellung des Bearbeitungswerkzeuges besonders einfach, insbesondere wenn die Schneide aus einer Vielzahl von Schneidelementen aufgebaut ist.

Mit einer Weiterbildung kann das Schneidverhalten des Bearbeitungswerkszeuges weiterhin verbessert werden, da der Freiwinkel γ in Abhängigkeit vom Abstand zur Rotationsachse kontinuierlich angepaßt wird.

Eine Weiterbildung weist den Vorteil auf, daß das Bearbeitungswerkzeug im gesamten Schneidbereich eine im wesentlichen gleichmäßige Schneideigenschaft aufweist, da die maximale Vordringtiefe h_{c} der Schneide im gesamten Schneidumfang konstant ist, so daß sich die Schneideigenschaften des Bearbeitungswerkzeugs optimieren. Die maximale Vordringtiefe h_{c} der Schneide berechnet sich aus der Gleichung: h_{c} = 2rπ tanγ, woraus ersichtlich ist, daß eine indirekte Proportionalität zwischen dem Abstand der Schneide zur Drehachse und dem tanγ besteht, wobei der Freiwinkel γ zwischen 0° und 90° liegt, d.h., wenn der Abstand der Schneide zur Drehachse zunimmt, wird der Freiwinkel γ kleiner.

Durch eine Weiterbildung kann das Zerspannvolumen des Werkstoffs gemäß dem Radius angepaßt werden, wobei der Freiwinkel γ des Schneidelements und der Spanwinkel α des Schneidelements im Hinblick auf das zu bearbeitende Material in vorteilhafter Weise abgestimmt werden.

Darüber hinaus hat die Weiterbildung den Vorteil, daß sich die effektive Schneidlänge der Schneide erhöht, wobei beim Spanabtrag neben der Scherkraft in Drehrichtung noch eine zusätzliche Komponente der Scherkraft nach außen auftritt, so daß der Materialabtrag mit einem geringeren Kraftaufwand möglich ist.

Eine Weiterbildung weist den Vorteil auf, daß das Bearbeitungswerkzeug während des Bearbeitungsvorgangs dadurch stabilisiert wird, daß mehrere Schneiden gleichzeitig im Eingriff sind, so daß das Bearbeitungswerkzeug kaum von der gewünschten Drehachse abweicht. Ferner werden somit die Vibrationen des Bearbeitungswerkzeugs vermindert, so daß sich die Oberflächenbeschaffenheit der erzeugten Vertiefung verbessert.

Durch eine Weiterbildung kommt es zu einer vorteilhaften Stabilisierung und Vibrationsdämpfung, wobei die Herstellung des Bearbeitungswerkzeugs kostengünstig ist. Ferner sind zumindest drei Schneiden im gleichen Umfang wirksam, um die Stabilität der Konstruktion zu gewährleisten.

Ferner gewährleistet eine Weiterbildung, daß die Kerben als Spannbrecher dienen, so daß die Gefahr des Festfahrens des Bearbeitungswerkzeuges im Werkstück vermindert wird.

Darüber hinaus hat die Weiterbildung den Vorteil, daß das Bearbeitungswerkzeug durch ein Führungselement mit Schneidfunktionen geführt wird, wenn es sich mit dem Werkstück in Kontakt befindet, wodurch Schwingungen zwischen dem Werkstück und dem Bearbeitungswerkzeug vermindert werden. Ferner wird das Bearbeitungswerkzeug gegen mechanische Deformationen in vorteilhafter Weise versteift, so daß die Form des Bearbeitungswerkzeugs während des Bearbeitungsvorgangs nicht negativ beeinträchtigt wird.

Mit einer Weiterbildung wird das Bearbeitungswerkzeug während des Bearbeitungsvorgangs über die gesamte Mantelfläche geführt, da das spiralförmige Führungselement die gesamte Mantelfläche bei einer Drehung durchläuft.

Durch die Weiterbildung erhält man eine zusätzliche Radial- bzw. Spiralschneide, welche die Schneidleistung des Bearbeitungswerkzeuges weiter erhöht, da sich der effektive Materialabtrag und der Abtransport des Spanns weiter erhöht. Insbesondere ist es vorteilhaft, wenn der Freiwinkel γ der Spiralschneide, welcher zwischen der Mantelfläche und der dem Werkstück zugewandten Schneidfläche des Schneidelements ausgebildet ist, vom Pol der Mantelfläche in Richtung des Äquators der Mantelfläche abnimmt. Ferner kann das Bearbeitungswerkzeug materialsparend hergestellt werden, wobei sich die Öffnungen der Schneiden und die Spiralöffnung derart überlappen, daß die Mantelfläche des Bearbeitungswerkzeugs im wesentlichen lediglich aus den Schneiden und der Spiralschneide aufgebaut ist.

Mit einer Weiterbildung wird das Bearbeitungswerkzeug zusätzlich gegen mechanische Verformungen versteift, da die Schneiden von der Spirale mehrfach gestützt werden. Ferner dienen die Schnittpunkte der Spirale mit den Schneiden als Spanbrecher.

Weitere Einzelheiten, Merkmale und Vorteile der Erfindung ergeben sich aus der nachfolgenden Beschreibung einer vorteilhaften Ausführungsform der Erfindung anhand der Zeichnungen.

Es zeigen:
- Fig. 1: eine perspektivische Ansicht einer Ausführungsform eines Bearbeitungswerkzeug gemäß der Erfindung;
- Fig. 2: eine Seitenansicht der Ausführungsform von Figur 1;
- Fig. 3: eine Schnittansicht eines Schneidelements im Bereich des Pols der Mantelfläche des Bearbeitungswerkzeug von Fig. 1 und 2;
- Fig. 4: eine Schnittansicht des Schneidelements im Bereich des Äquators der Mantelfläche des Bearbeitungswerkzeug von Fig. 1 und 2; und
- Fig. 5: eine graphische Darstellung von Schneidleistungen in bezug auf den Durchmesser von Bearbeitungswerkzeugen.

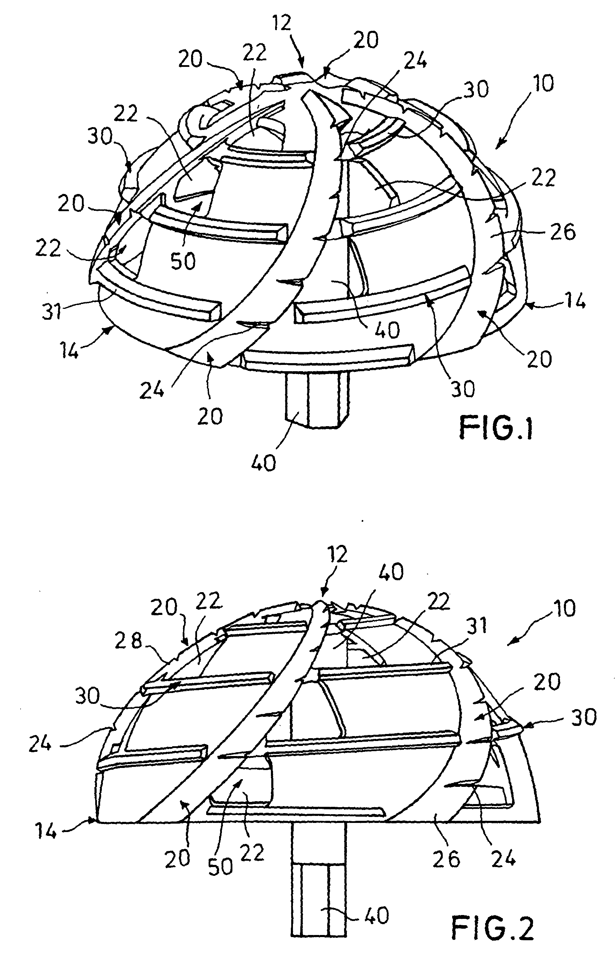

In Figur 1 ist eine vorteilhafte Ausführungsform eines chirurgischen Bearbeitungswerkzeugs gemäß der vorliegenden Erfindung dargestellt, welche einen halbkugelförmigen Werkzeugkopf 10 in Form einer halbkugelförmigen Mantelfläche 10 und einen dadurch umgrenzten hohlen Innenbereich 50 aufweist. An der halbkugelförmigen Mantelfläche 10 sind fünf Schneiden 20 angeordnet, welche sich vom Pol 12 der Mantelfläche 10 zum Äquator 14 der Mantelfläche 10 erstrecken, wobei die Schneiden 20 gleichmäßig über der Mantelfläche 10 verteilt sind und spiralförmig gekrümmt sind, so daß die Schneiden 20 die Form eines Propellers abbilden. Die einzelnen Schneiden 20 sind jeweils einstückig ausgebildet, so daß die Schneiden 20 ein Schneidelement aufweisen. Die gesamte Schnittfläche wird durch eine Überlappung der einzelnen Schneiden 20 im Rotationsbild von ca. 5% bis 15% erreicht. Ferner ist ein zusätzliches Führungselement 30 in Form einer Spirale an der Mantelfläche 10 ausgebildet, die sich im wesentlichen vom Pol 12 bis zum Äquator 14 spiralförmig erstreckt, wobei das Führungselement 30 im Bereich der Schneiden 20 unterbrochen ist und wobei das Führungselement 30 den Bereich zwischen zwei Schneiden 20 zumindest zweimal durchläuft.

Zum Abtransport des Spans in den hohlen Innenbereich 50 sind Öffnungen 22 in der Mantelfläche 10 zwischen den Schneiden 20 dem Führungselement 30 ausgebildet. Beim Bearbeitungsvorgang dreht sich das dargestellte Bearbeitungswerkzeug entgegen dem Uhrzeigersinn, wobei jede Schneide 20 eine Vielzahl von Kerben 24 aufweist, die als Spanbrecher dienen, so daß die von den Schneiden 20 zerteilten Späne eines nicht dargestellten Werkstücks durch die Öffnungen 22 in den hohlen Innenbereich 50 abtransportiert werden, wobei die Länge der Kerben 24 vom Pol 12 zum Äquator 14 zunimmt. Ferner ist ein sich durch den hohlen Innenbereich 50 erstreckender Lagerschaft 40 an der Mantelfläche 10, insbesondere am Pol 12, befestigt, welcher sowohl die Drehachse als auch die Symmetrieachse des Bearbeitungswerkzeugs bildet. Der Lagerschaft 40 ist teilweise durch Öffnungen 22 in dem hohlen Innenbereich 50 zu sehen.

In Fig. 2 ist eine Seitenansicht der Ausführungsform des Bearbeitungswerkzeuges von Figur 1 dargestellt, wobei insbesondere die Form der halbkugelförmigen Mantelfläche 10 ersichtlich ist.

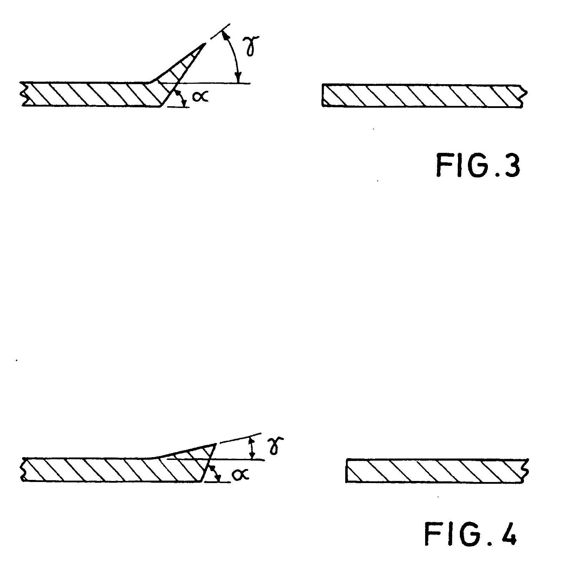

In Fig. 3 ist eine Schnittansicht eines Schneidelements 20 im Bereich des Pols der Mantelfläche 10 des erfindungsgemäßen Bearbeitungswerkzeuges von Fig. 1 und 2 dargestellt. Zwischen der Mantelfläche 10 und der dem Werkstück zugewandten Schneidfläche 26 des Schneidelements bzw. der Schneide 20 wird ein Freiwinkel γ ausgebildet. Andererseits bildet sich zwischen der Mantelfläche 10 und der dem Werkstück abgewandten Schneidfläche 28 des Schneidelements bzw. der Schneide 20 ein Spanwinkel α.

Darüber hinaus ist der Freiwinkel γ und der Spanwinkel α des Schneidelements 20 in Fig. 4 im Bereich des Äquators 14 der Mantelfläche 10 der vorteilhaften Ausführungsform des Bearbeitungswerkzeug von Fig. 1 und 2 dargestellt. Im Vergleich zu Fig. 3 ist der Freiwinkel γ in Fig. 4 kleiner, woraus ersichtlich wird, daß sich der Spanabtrag der Schneide 20 vom Pol 12 der Mantelfläche 10 in Richtung des Äquators 14 der Mantelfläche 10 aufgrund der Abnahme des Freiwinkels γ vermindert.

Andererseits ist der Spanwinkel α in Fig. 4 im Vergleich zu Fig. 3 größer, um das Zerspannvolumen des Werkstoffs gemäß dem Radius anzupassen. Somit nimmt der Spanwinkel α der Schneide 20, welcher zwischen der Mantelfläche 10 und der dem Werkstück abgewandten Schneidfläche 28 des Schneidelements bzw. der Schneide 20 ausgebildet ist, vorteilhafterweise vom Pol 12 der Mantelfläche 10 in Richtung des Äquators 14 der Mantelfläche 10 zu.

Ferner ist aus Fig. 2 ersichtlich, daß der Freiwinkel γ der Schneide 20 vom Pol 12 der Mantelfläche 10 in Richtung des Äquators 14 der Mantelfläche 10 kontinuierlich abnimmt, da die Schneide 20 vom Pol 12 der Mantelfläche 10 in Richtung des Äquators 14 der Mantelfläche 10 stetig breiter wird.

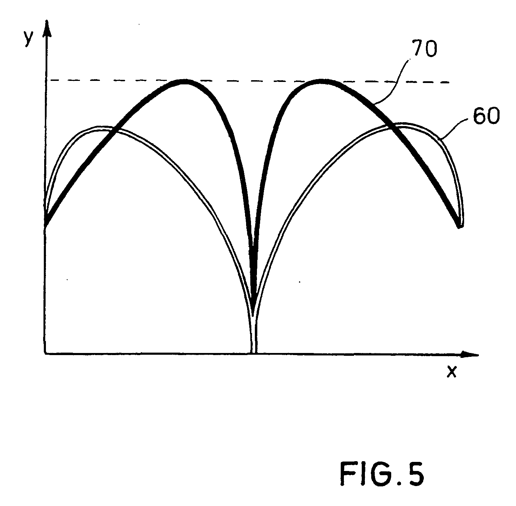

In Fig. 5 ist eine graphische Darstellung von Schneidleistungen in bezug auf den Durchmesser von Bearbeitungswerkzeugen gezeigt, wobei die x-Achse den Durchmesser von Bearbeitungswerkzeugen und die y-Achse die Schneidleistung von Bearbeitungswerkzeugen angibt. Eine Kurve 70 zeigt den Verlauf der Schneidleistung eines erfindungsgemäßen Bearbeitungswerkzeugs über den Durchmesser, wohingegen eine Kurve 60 den Verlauf der Schneidleistung eines herkömmlichen Bearbeitungswerkzeugs über dem Durchmesser zeigt, wobei die gestrichelte Linie die Schneidleistung von 100% angibt. Der Schnittpunkt der Kurven 60, 70 mit der x-Achse zeigt den Pol 12 der Bearbeitungswerkzeuge an, wobei die Schnittgeschwindigkeit im Pol 12 der Bearbeitungswerkzeuge Null beträgt, da der Abstand zur Drehachse im Pol 12 Null beträgt.

Aus der graphischen Darstellung von Fig. 5 ist ersichtlich, daß die Schneidleistung des erfindungsgemäßen Bearbeitungswerkzeugs gegenüber dem herkömmlichen Bearbeitungswerkzeugs größer ist, da die Fläche unter der Kurve 70 größer ist. Ferner wird durch die Abnahme des Freiwinkels γ der Schneide 20 zum Rand bzw. Pol 12 des Bearbeitungswerkzeugs hin die Tendenz des Verlaufens des erfindungsgemäßen Bearbeitungswerkzeuges gegenüber herkömmlichen Bearbeitungswerkzeugen vermindert, da die maximale Schneidleistung des erfindungsgemäßen Bearbeitungswerkzeuges gegenüber herkömmlichen Bearbeitungswerkzeugen näher am Pol 12 der Mantelfläche 10 angeordnet ist. Dies führt dazu, daß das erfindungsgemäße Bearbeitungswerkzeug gegenüber herkömmlichen Bearbeitungswerkzeugen bei entsprechendem Vorschub in Richtung der Drehachse während des Bearbeitungsvorgangs selbstzentrierend wirkt.

Darüber hinaus weist das Führungselement bei einer weiteren nicht gezeigten Ausführungsform eines Bearbeitungswerkzeugs gemäß der Erfindung, wobei lediglich die Unterschiede zur in den Fig. 1 bis 4 dargestellten Ausführungsform erläutert werden, eine Radial- bzw. Spiralschneide und eine damit kooperierende Radial- bzw. Spiralöffnung in der Mantelfläche 10 zum Abtransport des Spans in den hohlen Innenbereich 50 auf, wobei insbesondere der Freiwinkel γ der Spiralschneide, welcher zwischen der Mantelfläche 10 und der dem Werkstück zugewandten Schneidfläche des Schneidelements ausgebildet ist, vom Pol 12 der Mantelfläche 10 in Richtung des Äquators 14 der Mantelfläche 10 abnimmt. Ferner schneidet die Spiralschneide die auf der Mantelfläche verteilten propellerförmigen Schneiden 20 mehrfach, insbesondere zumindest zweimal, wobei die Schnittpunkte der Spiralschneide mit den propelierförmigen Schneiden 20 als Spanbrecher dienen. Des weiteren überlagern sich die Öffnungen 22 der propellerförmigen Schneiden 20 mit der Spiralöffnung, so daß die Mantelfläche 10 des Bearbeitungswerkzeugs lediglich aus der Spiralschneide und den propellerförmigen Schneiden 20 aufgebaut ist, wobei die propellerförmigen Schneiden 20 im Bereich des Äquators 14 zur Stabilisierung insbesondere durch einen Steg verbunden sind.

Gemäß einer weiteren nicht gezeigten Ausführungsform sind drei Schneiden über die Mantelfläche verteilt, wobei die einzelnen Schneiden aus Schneidelementen aufgebaut sind, welche zwischen konzentrischen Kreisen der Mantelfläche angeordnet sind und eine gekrümmte Form aufweisen.

Wie die vorstehende Beschreibung zeigt, können vielfache Abwandlungen und Abänderungen von der dargestellten Ausgangsform vorgenommen werden, ohne den Rahmen der Erfindung zu verlassen. So kann beispielsweise die sich vom Pol der Mantelfläche zum Äquator der Mantelfläche erstreckende Schneide auch durch eine Vielzahl von unabhängigen Schneidelementen aufgebaut sein. Ferner kann ein erfindungsgemäßes Bearbeitungswerkzeug fest eingespannt sein, wobei das Werkstück gedreht wird. Darüber hinaus kann die Mantelfläche des Bearbeitungswerkzeugs eine Form aufweisen, die keiner vollkommenen Halbkugel entspricht, sondern einer Halbkugel ähnlich ist oder einem Teil einer Halbkugel entspricht, der sich nicht bis zum Äquator erstreckt. Des weiteren kann das Führungselement aus an der Mantelfläche angeordneten Kreiselementen oder einer Vielzahl von einzelnen Segmenten aufgebaut sein.

## Patentansprüche

1. Chirurgisches Bearbeitungswerkzeug zur Schaffung einer Vertiefung in einem Knorpelund/oder Knochengewebe für eine Gelenksprothese, insbesondere in einem Hüftgelenksknochen für eine Pfannenprothese, welches eine im wesentlichen halbkugelförmige Mantelfläche (10) und einen dadurch umgrenzten hohlen Innenbereich (50) aufweist, wobei an der Mantelfläche (10) zumindest ein sich vom Pol (12) der Mantelfläche (10) zum Äquator (14) des Mantelfläche (10) erstreckendes Schneidelement (20) mit einer dem Werkstück zugewandten Schneidfläche (26) und einer dem Werkstück abgewandten Schneidfläche (28) vorgesehen ist sowie eine damit kooperierende Öffnung (22) in der Mantelfläche (10) zum Abtransport des Spans in den hohlen Innenbereich (50) ausgebildet ist, **dadurch gekennzeichnet, daß** ein Freiwinkel γ des Schneidelementes (20), welcher zwischen der Mantelfläche (10) und der dem Werkstück zugewandten Schneidfläche (26) ausgebildet ist, vom Pol (12) der Mantelfläche (10) in Richtung des Äquators (14) der Mantelfläche (10) abnimmt.

2. Chirurgisches Bearbeitungswerkzeug nach Anspruch 1, **dadurch gekennzeichnet, daß** der Freiwinkel γ in Abhängigkeit vom Abstand zur Drehachse stufenweise abnimmt.

3. Chirurgisches Bearbeitungswerkzeug nach Anspruch 1, **dadurch gekennzeichnet, daß** der Freiwinkel γ in Abhängigkeit vom Abstand zur Drehachse kontinuierlich abnimmt.

4. Chirurgisches Bearbeitungswerkzeug nach einem der Ansprüche 1 bis 3, **dadurch gekennzeichnet, daß** sich der Freiwinkel γ aus arctan h_{c} / 2rπ berechnet, wobei die maximale Vordringtiefe h_{c} der Schneide (20) in ein Werkstück über den gesamten Schneidumfang im wesentlichen konstant ist.

5. Chirurgisches Bearbeitungswerkzeug nach einem der Ansprüche 1 bis 4, **dadurch gekennzeichnet, daß** ein Spanwinkel α des Schneidelementes (20), welcher zwischen der Mantelfläche (10) und der dem Werkstück abgewandten Schneidfläche (28) ausgebildet ist, vom Pol (12) der Mantelfläche (10) in Richtung des Äquators (14) der Mantelfläche (10) zunimmt.

6. Chirurgisches Bearbeitungswerkzeug nach einem der Ansprüche 1 bis 5, **dadurch gekennzeichnet, daß** die Schneide (20) spiralförmig gekrümmt ist.

7. Chirurgisches Bearbeitungswerkzeug nach einem der Ansprüche 1 bis 6, **dadurch gekennzeichnet, daß** sich zwei bis sieben Schneiden (20) vom Pol (12) in Richtung des Äquators (14) erstrecken, die gleichmäßig über der Mantelfläche (10) verteilt sind.

8. Chirurgisches Bearbeitungswerkzeug nach Anspruch 7, **dadurch gekennzeichnet, daß** sich drei bis fünf Schneiden (20) vom Pol (12) in Richtung des Äquators (14) erstrecken, die gleichmäßig über der Mantelfläche (10) verteilt sind.

9. Chirurgisches Bearbeitungswerkzeug nach einem der Ansprüche 1 bis 8, **dadurch gekennzeichnet, daß** die Schneiden (20) Kerben (24) aufweisen.

10. Chirurgisches Bearbeitungswerkzeug nach einem der Ansprüche 1 bis 9, **dadurch gekennzeichnet, daß** zumindest ein zusätzliches Führungselement (30) mit Schneidfunktionen radial zu der Mantelfläche (10) verlaufend ausgebildet ist.

11. Chirurgisches Bearbeitungswerkzeug nach Anspruch 10, **dadurch gekennzeichnet, daß** sich das Führungselement (30) im wesentlichen vom Pol (12) bis zum Äquator (14) spiralförmig erstreckt.

12. Chirurgisches Bearbeitungswerkzeug nach einem der Ansprüche 10 oder 11, **dadurch gekennzeichnet, daß** das Führungselement (30) eine Radialschneide und eine damit kooperierende Radialöffnung in der Mantelfläche (10) zum Abtransport des Spans in den hohlen Innenbereich (50) aufweist.

13. Chirurgisches Bearbeitungswerkzeug nach einem der Ansprüche 10 bis 13 wenn abhängig von 7, **dadurch gekennzeichnet, daß** das Führungselement (30) jede der zwei bis sieben Schneiden (20) zumindest zweimal schneidet.

14. Chirurgisches Bearbeitungswerkzeug nach einem der vorhergehenden Ansprüche, **dadurch gekennzeichnet, daß** zur Drehung um die die Drehachse bildende Symmetrieachse ein sich durch den hohlen Innenbereich (50) erstreckender Lagerschaft (40) an der Mantelfläche (10) befestigt ist.

## Claims

1. Surgical tool for creating a recess in a cartilage and/or bone tissue for a joint prosthesis, particularly in a hip joint bone for a socket prosthesis, which comprises a substantially hemisperical superficies (10) and a hollow inner area (50) enclosed thereby, said superficies (10) being provided with a cutting element (20) extending from the pole (12) of said superficies (10) to the equator (14) of said superficies (10) and comprising a cutting surface (26) facing the workpiece and a cutting surface (28) facing away from the workpiece, as well as an opening (22) cooperating therewith being formed in said superficies (10) for taking away chip into said hollow inner area (50), **characterized in that** a clearance angle γ of said cutting element (20), said angle γ being formed between said superficies (10) and said cutting surface (26) facing the workpiece, decreases from said pole (12) of said superficies (10) in the direction towards said equator (14) of said superficies (10).

2. Surgical tool according to claim 1, **characterized in that**, dependent on the distance from the axis of rotation, said clearance angle γ decreases in stages.

3. Surgical tool according to claim 1, **characterized in that**, dependent on the distance from the axis of rotation, said clearance angle γ decreases continously.

4. Surgical tool according to anyone of claims I to 3, **characterized in that** said clearance angle γ is calculated from arctan (h_{c} / 2rπ), wherein the maximum depth of advance h_{c} of said cutting edge (20) into a workpiece is substantially constant over the entire cutting circumference.

5. Surgical tool according to anyone of claims 1 to 4, **characterized in that** a rake angle α of said cutting element (20), which is formed between said superficies (10) and said cutting surface (28) facing away from the workpiece, increases from said pole (12) of said superficies (10) to said equator (14) of said superficies (10).

6. Surgical tool according to anyone of claims 1 to 5, **characterized in that** said cutting edge (20) is spirally arcuate.

7. Surgical tool according to anyone of claims 1 to 6, **characterized in that** two to seven cutting edges (20) extend from said pole (12) towards said equator (14), wherein said cutting edges (20) are uniformly distributed over said superficies (10).

8. Surgical tool according to claim 7, **characterized in that** three to five cutting edges (20) extend from said pole (12) towards said equator (14), wherein said cutting edges (20) are uniformly distributed over said superficies (10).

9. Surgical tool according to anyone of claims 1 to 8, **characterized in that** said cutting edges (20) comprise notches (24).

10. Surgical tool according to anyone of claims 1 to 9, **characterized in that** at least one additional guiding element (30) having cutting functions is formed extending radially from said superficies (10).

11. Surgical tool according to claim 10, **characterized in that** said guiding element (30) substantially extends spirally from said pole (12) to said equator (14).

12. Surgical tool according to claim 10 or 11, **characterized in that** said guiding element (30) comprises a radial cutting edge and a radial opening in said superficies (10) cooperating therewith to take away the chip into said hollow inner area (50).

13. Surgical tool according to anyone of claims 10 to 13 if dependant of claim 7, **characterized in that** said guiding element (30) intersects at least twice with each of the two to seven cutting edges (20).

14. Surgical tool according to anyone of the preceding claims, **characterized in that** for rotation around the axis of symmetry which forms the axis of rotation, a bearing shaft (40) extending through said hollow inner area (50) is attached on said superficies (10).

## Revendications

1. Outil de traitement chirurgical destiné à pratiquer une cavité dans un cartilage et / ou un tissu osseux pour une prothèse articulaire, en particulier dans un os iliaque pour une prothèse cotyloïde, lequel outil comporte sensiblement une surface de recouvrement de forme hémisphérique (10) et une zone interne creuse (50) en suivant le contour, sachant qu'il est prévu que sur la surface de recouvrement (10) au moins un élément de coupe (20) s'étendant depuis le pôle (12) de la surface de recouvrement (10) jusqu'à la ligne équinoxiale (14) de la surface de recouvrement (10), avec une surface de coupe (26) tournée vers la pièce à usiner et une surface de coupe (28) détournée de la pièce à usiner ainsi qu'une ouverture (22) en rapport qui est ainsi formée dans la surface de recouvrement (10) destinée à l'évacuation de l'éclat dans la zone creuse interne (50), **caractérisé en ce qu'**il est réalisé un angle libre γ avec l'élément de coupe (20), lequel est formé entre la surface de recouvrement (10) et la surface de coupe (26) tournée vers la pièce à usiner, va en diminuant du pôle (12) de la surface de recouvrement (10) en direction de la ligne équinoxiale (14) de la surface de recouvrement (10).

2. Outil de traitement chirurgical selon la revendication 1, **caractérisé en ce que** l'angle libre γ diminue progressivement en fonction de la distance par rapport à l'axe de rotation.

3. Outil de traitement chirurgical selon la revendication 1, **caractérisé en ce que** l'angle libre γ diminue continuellement en fonction de la distance par rapport à l'axe de rotation.

4. Outil de traitement chirurgical selon l'une des revendications 1 à 3, **caractérisé en ce que** l'angle libre γ se calcule à partir de l'arctan h_{c} / 2rπ, moyennant quoi la profondeur maximale de pénétration he de la coupe (20) dans une pièce à usiner est, pour l'essentiel, constante par rapport à la totalité du volume de coupe.

5. Outil de traitement chirurgical selon l'une des revendications 1 à 4, **caractérisé en ce qu'**un angle de dégagement α de l'élément de coupe (20), lequel est formé entre la surface de revêtement (10)
et la surface de coupe (28) disposée sur la pièce à usiner, diminue depuis le pôle (12) de la surface de recouvrement (10) en direction de la ligne équinoxiale (14) de la surface de recouvrement (10).

6. Outil de traitement chirurgical selon l'une des revendications 1 à 5, **caractérisé en ce que** la coupe (20) est découpée en forme de spirale.

7. Outil de traitement chirurgical selon l'une des revendications 1 à 6, **caractérisé en ce que** de deux à sept coupes (20) s'étendent depuis le pôle (12) en direction de la ligne équinoxiale (14) et sont réparties régulièrement au-dessus de la surface de recouvrement (10).

8. Outil de traitement chirurgical selon la revendication 7, **caractérisé en ce que** de trois à cinq coupes (20) s'étendent depuis le pôle (12) en direction de la ligne équinoxiale (14) et sont réparties régulièrement au-dessus de la surface de recouvrement (10).

9. Outil de traitement chirurgical selon l'une des revendications 1 à 8, **caractérisé en ce que** les coupes (20) comportent des encoches (24).

10. Outil de traitement chirurgical selon l'une des revendications 1 à 9, **caractérisé en ce qu'**au moins un élément de guidage supplémentaire (30) est formé en s'étendant avec les fonctions de coupe en direction radiale par rapport à la surface de recouvrement (10).

11. Outil de traitement chirurgical selon la revendication 10, **caractérisé en ce que** l'élément de guidage supplémentaire (30) s'étend pour l'essentiel, en forme de spirale, depuis le pôle (12) jusqu'à la ligne équinoxiale (14).

12. Outil de traitement chirurgical selon l'une des revendications 10 ou 11, **caractérisé en ce que** l'élément de guidage (30) comporte une coupe radiale et une ouverture radiale servant ainsi dans la surface de recouvrement (10) à l'évacuation de l'éclat dans la zone interne creuse (50).

13. Outil de traitement chirurgical selon l'une des revendications 10 à 13, si dépendant de 7, **caractérisé en ce que** l'élément de guidage (30) coupe au moins deux fois chacune des deux à sept coupes (20).

14. Outil de traitement chirurgical selon l'une des revendications précédentes, **caractérisé en ce que** pour faire pivoter l'axe de symétrie formant l'axe de rotation, une tige-support (40) s'étendant à travers la zone interne creuse (50) est fixée sur la surface de recouvrement (10).
